(19) Europäisches Patentamt
European Patent Office
Office européen des brevets

(11) Numéro de publication: **0 242 302**
**B1**

(12) **FASCICULE DE BREVET EUROPEEN**

(45) Date de publication du fascicule du brevet: **31.10.90**

(21) Numéro de dépôt: **87400878.2**

(22) Date de dépôt: **16.04.87**

(51) Int. Cl.$^5$: **C 07 K 3/18,** B 01 J 20/22, B 01 D 15/08, A 61 K 39/10, C 07 K 3/20

Netherlands Patent Office
Library tel. 070 - 3986655
fax 070 - 3900190 Rijswijk

(54) **Nouveau matériau pour chromatographie d'affinité et son application à la séparation et à la purification des antigènes protéiques des bactéries du genre Bordetella.**

(30) Priorité: **16.04.86 FR 8605457**

(43) Date de publication de la demande:
**21.10.87 Bulletin 87/43**

(45) Mention de la délivrance du brevet:
**31.10.90 Bulletin 90/44**

(84) Etats contractants désignés:
**AT BE CH DE ES GB GR IT LI LU NL SE**

(56) Documents cités:
**EP-A-0 003 916**
**EP-A-0 140 386**
**EP-A-0 175 841**

**BIOLOGICAL ABSTRACTS, vol. 58, no. 12, 15 décembre 1974, page 6941, résumé no. 64553, Philadelphia, US; R.L. HUDGIN et al.: "The isolation and properties of a rabbit liver binding protein specific for asialoglycoproteins" & J. BIOL. CHEM. 249(17): 5536-5543, ILLUS. 1974**

(73) Titulaire: **INSTITUT MERIEUX Société anonyme dite:**
**17, rue Bourgelat**
**F-69002 LYON (FR)**

(72) Inventeur: **Quentin-Millet, Marie-José**
**70, Cours Emile Zola**
**F-69100 Villeurbanne (FR)**
Inventeur: **Arminjon, François**
**17, Boulevard de la Croix-Rousse**
**F-69004 Lyon (FR)**

(74) Mandataire: **Nony, Michel et al**
**Conseil en Brevets d'Invention 29 rue Cambacérès**
**F-75008 Paris (FR)**

(56) Documents cités:
**CHEMICAL ABSTRACTS, vol. 74, no. 19, 10 mai 1971, page 187, résumé no. 96321g, Columbus, Ohio, US; A.G. MORELL et al.: "Role of sialic acid in determining the survival of glycoproteins in the circulation" & J. BIOL. CHEM. 1971, 246(5), 1461-7**

**BBRC, vol. 122(3), 1984, pp 938-944 .**

**EP 0 242 302 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention a pour objet l'utilisation, comme ligand de chromatographie d'affinité pour la toxine pertussis, de la fétuine ou de l'haptoglobine désialylée, en vue d'effectuer notmament la séparation et la purification des antigènes protéiques des bactéries appartenant au genre Bordetella.

On sait que, la culture de bactéries du genre Bordetella, par exemple Bordetella pertussis, Bordetella parapertussis et Bordetella bronchiseptica, permet l'obtention de la protéine F-HA (Filamentous hémagglutinin). De plus, la culture de B. pertussis permet également l'obtention d'une exotoxine protéique: la toxine pertussis, encore appelée LPF ou LPF-HA (Leukocytosis Promoting Factor-Hemagglutinin). Ces protéines peuvent être utilisées notamment dans la préparation de vaccins acellulaires contre la coqueluche.

La séparation et la purification des antigènes protéiques des bactéries du genre Bordetella pose des problèmes techniques difficiles à résoudre pour obtenir les antigènes purifiés avec de bons rendements.

Généralement on utilise, lors de la purification, des techniques de chromatographie d'affinité.

Dans la demande de brevet britannique No. 2.015.531 on a préconisé l'utilisation d'un procédé de chromatographie d'affinité à l'aide d'un support polymérique insoluble sur lequel est fixée une sialoprotéine ou une autre substance riche en acide sialique. La sialoprotéine permet de fixer sélectivement la toxine pertussis qui peut ensuite être éluée.

Dans la demande de brevet européen No. 0140386 on a décrit une méthode de chromatographie utilisant comme ligand sélectif pour la toxine pertussis une céruloplasmine dénaturée par la chaleur.

On a maintenant découvert que, de façon surprenante, la toxine pertussis a davantage d'affinité pour les glycoprotéines désialylées immobilisées sur un support que pour les sialoprotéines, comme cela sera montré ci-après. Ce résultat est d'autant plus surprenant que, dans le cas où c'est la toxine pertussis qui est immobilisée sur un support, on avait observé que la fétuine inhibe davantage la fixation de la fétuine marquée à l'iode 125 que l'asialofétuine (93% d'inhibition seulement par rapport à la fétuine; voir R. D. Sekura et al., "Pertussis Toxin Structural Elements involved in the interaction with cells", Proceeding of the Pertussis Toxin conference, Bethesda, Sept. 20—21, 1984 edited by R. D. Sekura, J. Moss and M. Vaughan 1985, Academic Press Inc., pages 45—64.

L'asialofétuine a déjà été décrite comme ligand de chromatographie d'affinité, sous la forme d'asialofétuine-Sépharose®, pour extraire une lectine d'un homogénat humain; voir J. Hirabayashi et al., Biochem. Biophys. Res. Comm. Vol. 122, No. 3, 938—944 (1984).

L'invention a pour objet l'utilisation, comme ligand de chromatographie d'affinité pour la toxine pertussis, d'une glycoprotéine désialylée choisie parmi la fétuine désialylée et l'haptoglobine désialylée.

On sait que la fétuine et l'haptoglobine contiennent des groupements oligo-osidiques sialylés; voir par exemple J. U. Baenziger et al, The Journal of Biological Chemistry, vol. 254, No. 3, pp. 789—795 (1979) et B. Nilsson et al, Proceedings of VI International Symposium on glycoconjugates (1981), pages 275—276.

L'hydrolyse acide de la fétuine ou de l'haptoglobine selon les méthodes connues, convertit les structures osidiques sialylées en structures osidiques désialylées correspondantes, et on obtient ainsi l'asialofétuine ou l'asialo-haptoglobine.

La fétuine et l'haptoglobine sont des produits commerciaux vendus notamment par la Société SIGMA.

Ces protéines sont soumises, avant ou éventuellement après couplage avec le support solide, à un traitement de désialylation. Cette désialylation est effectuée par hydrolyse douce selon les méthodes connues; voir par exemple Spiro R. G. et al, J. Biol. Chem. 1974, *249*, 5704—5717.

Le support solide sur lequel est couplée la protéine désialylée peut être tout support solide classique utilisé habituellement en chromatographie d'affinité.

Le support est par exemple un support polymérique constitué par un dérivé polyosidique, un polyacrylamide, un polymère ou copolymère contenant des motifs N-acryloyl-2-amino-2-hydroxyméthyl-1,3-propanediol tel que les supports vendus par I.B.F. (Institut Biologique Français) sous les désignations commerciales Trisacryl®.

Parmi les dérivés polyosidiques utilisables on citera en particulier la cellulose, de dextran, l'agarose, le Sépharose®, l'amidon, ainsi que les dérivés polyosidiques modifiés par éthérification avec des dérivés d'alcool aminé. Ces dérivés polyosidiques modifiés répondent à la formule schématique

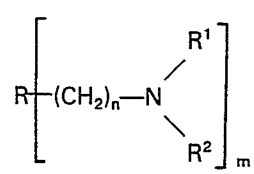

dans laquelle R représente le reste du polyoside, n est un nombre entier pouvant varier de 1 à 10 et de préférence de 2 à 5, $R_1$ et $R_2$ représente un radical alkyle inférieur ou un radical alkyle inférieur hydroxylé et m représente le nombre de groupements éthérifiants fixés sur la molécule polyosidique.

Parmi ces dérivés polyosidiques modifiés on citera par exemple la diéthylaminoéthyl cellulose (DEAE cellulose), le DEAE dextran, le DEAE amidon, etc.

Les polymères utilisés comme supports peuvent être eux-mêmes fixés sur un support minéral poreux constitué par exemple par un oxyde

métallique tel que la silice, l'alumine, la magnésie, ou par des dérivés synthétiques ou naturels de ces oxydes tels que des verres, des silicates, des borosilicates, le kaolin.

Lesdits polymères peuvent être fixés sur le support minéral poreux par imprégnation, le revêtement de polymère étant ensuite, si nécessaire, stabilisé par réticulation selon des méthodes connues. L'agent réticulant est par exemple un composé dicarbonylé, une halohydrine, un diépoxyde. Les polymères supports peuvent également être fixés sur les supports minéraux par l'intermédiaire d'agents de couplage bifonctionnels.

Les protéines désialylées peuvent aussi être fixées sur le support polymérique à l'aide d'un agent de couplage bifonctionnel approprié, selon les méthodes connues. Les agents de couplage sont par exemple des dérivés bifonctionnels tels que le bromure de cyanogène, des dialdéhydes, des diépoxydes.

Les protéines désialylées peuvent aussi être fixées directement sur un support minéral poreux.

Par exemple, dans le cas d'un support de silice, on prépare un dérivé aminoalkylsilane de la silice, et on peut alors fixer la protéine désialylée sur l'aminoalkylsilane à l'aide d'un agent bifonctionnel tel que le glutaraldéhyde; voir par exemple P. J. Robinson et al., Biochim. Biophys. Acta, *242*, 659—661 (1971).

Les protéines désialylées peuvent également être fixées sur des supports minéraux poreux selon la méthode décrite dans la demande de brevet français FR—A—2.403.098. Ce procédé consiste a revêtir un support minéral poreux d'un polymère polyosidique capable de donner lieu à la réaction de coupure oxydante des groupements glycol à l'aide d'agents oxydants tels que les periodates. On obtient alors un revêtement polycarbonylé et le ligand, par exemple la glycoprotéine désialylée, peut alors se fixer sur les groupements carbonylés formés. On peut ensuite, si désiré, réduire en amine le groupement imine formé.

L'invention a également pour objet un procédé de purification des antigènes protéiques des bactéries appartenant au genre Bordetella. Ce procédé consiste essentiellement à mettre en contact une solution contenant lesdits antigènes protéiques avec un support de chromatographie d'affinité tel que défini précédemment, puis à recueillir ou à éluer les antigènes recherchés.

On peut ainsi séparer la toxine pertussis qui se fixe sur le support de chromatographie d'affinité tandis que le F—HA reste en solution, puis éluer la toxine pertussis à l'aide d'un agent d'élution approprié.

On peut également utiliser le procédé de l'invention pour purifier une fraction enrichie en F—HA de façon à la débarrasser de la toxine pertussis qu'elle est susceptible de contenir, cette dernière étant retenue par le support de chromatographie d'affinité. En séparant la solution obtenue après mise en contact avec le matériau de chromatographie, on obtient donc une solution purifiée de F—HA.

La solution de départ à purifier peut être par exemple le surnageant de culture de bactéries du genre Bordetella ou une solution de toxine pertussis ou de F—HA partiellement purifiée.

Pour purifier la toxine pertussis sur le support de chromatographie d'affinité de l'invention, on utilise une solution de départ dont le pH a été ajusté à une valeur de 6—8, de préférence 7.

La quantité de support de chromatographie d'affinité utilisée est fonction du volume de surnageant initial et/ou de la concentration en toxine pertussis dans le milieu de culture ou dans la fraction utilisée comme produit de départ. Le contact est effectué en bain ou en colonne, à une température de 2—30°C.

Pour éluer la toxine pertussis fixée sur le support de chromatographie d'affinité, on peut utiliser par exemple une solution tampon contenant, à une concentration suffisante, des sels et/ou agents chaotropiques classiques, par exemple le chlorure de magnésium, ou bien un tampon carbonate ayant un molarité supérieure à 25 mM et dont le pH est de l'ordre de 8,3 à 11,6.

Les exemples suivants illustrent l'invention sans toutefois la limiter.

Exemple 1

Purification de toxine pertussis par chromatographie d'affinité sur Sépharose 4B-asialofétuine.

a) Adsorption de la toxine pertussis sur le gel de chromatographie d'affinité

Une fraction enrichie en toxine pertussis obtenue après centrifugation et concentration d'une suspension bactérienne de *Bordetella pertussis* phase I, cultivée en fermenteur de 30 litres est passée sur une colonne de 4 cm de diamètre contenant 120 ml de support de chromatographie Sépharose 4B couplé à l'asialofétuine, avec un débit de 6 ml/cm$^2$/heure.

Le Sépharose 4-B a été couplé à l'asialofétuine de la façon suivante.

30 g de Sépharose-4B activé au CNBr (Pharmacia) sont mis à gonfler dans 6 litres de HCl 1 mM pendant 15 minutes environ. Le gel est ensuite lavé 3 fois par 6 litres de HCl 1 mM. 400 ml d'une solution contenant 1 mg/ml d'asialofétuine, $NaHCO_3$ 0,1 M et NaCl 0,5 M sont ajoutés au gel.

Le mélange est laissé à réagir à +4°C pendant une nuit sous agitation douce. 125 ml d'une solution d'éthanolamine 5 M pH 8,0 sont ajoutés au mélange. Après 4 heures d'incubation à température ambiante, le gel est lavé successivement par 500 ml de tampon acétate de sodium 0,1 M pH 4,0 contenant NaCl 1 M puis par 500 ml de tampon Tris-HCl 50 mM pH 7,5 contenant NaCl 1 M. Ce cycle de lavage est répété trois fois.

Le gel est ensuite lavé trois fois par 500 ml de tampon Tris-HCl 50 mM pH 7,5 en présence d'un conservateur tel le merthiolate à une concentration de 1/10 000 (p/v).

Le gel de Sépharose 4B couplé à l'asialofétuine est conservé à +4°C dans un tampon tel qu'un

tampon Tris-HCl 50 mM pH 7,5 en présence d'un conservateur, le merthiolate par exemple.

L'asialofétuine utilisée comme ligand dans la chromatographie d'affinité a été obtenue de la façon suivante:

Une solution aqueuse de fétuine (fétuine type III, Sigma) est hydrolysée par $H_2SO_4$ 0,05 N pendant 1 heure à 80°C. Après hydrolyse, la solution est dialysée contre plusieurs bains d'eau distillée pendant 24 heures à +4°C afin d'éliminer les acides sialiques libres. La solution d'asialofétuine peut être concentrée par ultrafiltration à l'aide d'un système équipé de membranes dont le seuil de coupure est égal à 10 000.

L'élimination des acides sialiques est contrôlée par un dosage colorimétrique spécifique des acides sialiques sur la protéine après et avant hydrolyse.

b) Elution de la toxine pertussis

Le gel est lavé avec deux volumes de colonne de tampon Tris-HCl 50 mM pH 7,5, c'est-à-dire jusqu'à disparition complète de l'absorption UV à 278 nm, puis avec un volume de colonne de tampon Tris-HCl 50 mM pH 7,5 contenant NaCl 1 M. La toxine pertussis est éluée avec 400 ml de tampon carbonate 100 mM pH 9,6.

La densité optique et l'activité hémagglutinante des fractions collectées en sortie de colonne sont mesurées.

Les fractions contenant le principe actif, c'est-à-dire celles possédant une forte activité hémagglutinante non inhibée par le cholestérol, sont réunies.

La toxine pertussis est ensuite précipitée par le sulfate d'ammonium à une concentration finale correspondant à 70% de la saturation.

La toxine pertussis ainsi purifiée induit une forte lymphocytose et sensibilise les souris CFW à l'histamine à la dose de 0,04 µg/souris. La capacité de la toxine d'induire la formation de clusters sur des cellules CHO est caractérisée par une activité spécifique de l'ordre de 65 000 à 260 000 CPU/µg.

Les résultats de contrôles physico-chimiques et d'activités biologiques (dosages colorimétriques des éventuels contaminants, ADN, ARN, sucre; dosage du taux d'endotoxine, électrophorèse en milieu SDS ou en milieu acide) sont en faveur d'une préparation finale homogène renfermant un principe actif hautement purifié.

Les analyses de la teneur en toxine pertussis dans le surnageant de culture initial et dans le précipité final indiquent un rendement de purification supérieur à 90%.

Exemple 2

Purification de toxine pertussis par chromatographie d'affinité sur Sphérosil® DEAE dextran-asialofétuine.

a) Adsorption de la toxine sur le gel de chromatographie d'affinité

Le pH de la fraction obtenue après centrifugation et concentration d'une suspension de *Bordetella pertussis* phase I est ajusté à 7,0 par addition d'une solution d'acide chlorhydrique 5 N. La fraction est passée sur une colonne de 2 cm de diamètre contenant 60 ml de support de chromatographie Sphérosil DEAE dextran couplé à l'asialofétuine, avec un débit de 60 ml$\times$cm$^{-2}\times$heure$^{-1}$.

Le Sphérosil DEAE dextran (support constitué de billes de silice XOC 005 recouvertes avec un minimum de DEAE dextran réticulé) a été couplé à l'asialofétuine de la façon suivante:

Le Sphérosil DEAE dextran est soumis à une oxydation ménagée afin de créer des fonctions aldéhydiques capables de réagir avec des groupements aminés du ligand. L'asialofétuine en solution à 4 mg/ml dans le tampon phosphate 10 mM pH 8,0 est mis en contact avec le gel pendant 15 heures à température ambiante. Le gel est ensuite lavé par les solutions suivantes:

tampon phosphate 10 mM pH 8,0
glycocolle 20 g/l, NaCl 0,17 M
citrate de sodium 0,05 pH 2,8, HCl 0,1 N.

b) Elution de la toxine pertussis

Le gel est lavé par 120 ml de tampon Tris-HCl 50 mM pH 7,5 puis par 70 ml de tampon Tris-HCl 50 mM contenant du NaCl 1 M. La toxine pertussis est éluée par le tampon Tris-HCl 50 mM pH 7,5 contenant $MgCl_2$ 4 M. La densité optique à 278 nm et l'activité hémagglutinante des fractions collectées en sortie de colonne sont mesurées. Les fractions contenant le principe actif, c'est-à-dire celles possédant une activité hémagglutinante élevée non inhibée par le cholestérol, sont réunies.

Le mélange obtenu est dessalé par filtration sur gel sur une colonne de Séphadex® G-25 (7,5×45 cm, Pharmacia) équilibrée avec le tampon Tris-HCl 50 mM pH 7,5. L'élution est réalisée avec ce même tampon.

La densité optique à 278 nm et l'activité hémagglutinante des fractions collectées en sortie de colonne sont mesurées. Les fractions contenant la toxine pertussis, c'est-à-dire celles possédant une activité hémagglutinante élevée, sont réunies.

Le principe actif est précipité par le sulfate d'ammonium par addition lente sous agitation de 47 g de $(NH_4)_2SO_4$ par 100 ml de solution. L'analyse des propriétés biologiques et physico-chimiques montre que le produit obtenu est un principe actif hautement purifié.

Exemple 3

Elimination d'éventuelles traces de toxine pertussis dans une fraction enrichie en F—HA.

Les procédés classiques de purification du F—HA, comme celui décrit par exemple dans la publication de Sato Y., Cowell J. L., Sato H., Burstyn D. G. and Manclark C. R. "Separation and purification of the hemagluttinins from *Bordetella pertussis*" Infect. and Immun., 1983, *41*, 1, 313—320, comportent une étape de désorption du F—HA à partir d'un support chromatographique tel l'hydroxyapatite avec le tampon phosphate 100 mM pH 7,0 contenant NaCl 0,5 M.

La densité optique à 278 nm et l'activité hémagglutinante des fractions recueillies en sortie de la colonne d'hydroxyapatite sont mesurées. Les fractions contenant le F—HA, c'est-à-dire celle possédant une activité hémagglutinante élevée et inhibée par le cholestérol, sont regroupées.

Dans la présente invention, afin d'éliminer les dernières traces de toxine pertussis éventuellement présentes, le mélange des fractions contenant le F—HA est passé sur une colonne de Sépharose 4B-asialofétuine. Le F—HA est élué directement, alors que les éventuelles traces de toxine sont retenues sur la colonne.

Le F—HA est alors précipité par le sulfate d'ammonium en ajoutant lentement, sous agitation, 47 g de $(NH_4)_2SO_4$ pour 100 ml de solution.

Le F—HA ainsi purifié n'induit ni lymphocytose, ni sensibilisation à l'histamine à la dose de 100 µg/souris. A la concentration de 100 µg/ml, le F—HA ne provoque pas de modification dans l'aspect du tapis cellulaire constitué de cellules d'ovaires de hamster (CHO), c'est-à-dire la formation d'amas cellulaires dits "clusters" caractéristiques de la présence de toxine pertussis. Son activité spécifique hémagglutinante mesurée sur érythrocytes d'oie est de l'ordre de 250 000 à 500 000 unités HA/mg de protéine. Cette activité est totalement inhibée par le cholestérol.

Exemple 4

Etude comparative de la capacité de fixation de la toxine pertussis par les supports de chromatographie d'affinité à base de fétuine et d'asialofétuine.

Un gel de chromatographie d'affinité a été préparé par immobilisation d'asialofétuine sur du Sépharose-4B activité ou CNBr (Pharmacia) selon la technique décrite à l'exemple 1.

Parallèlement, de façon analogue, un gel de chromatographe d'affinité a été préparé avec de la fétuine.

La fixation de la fétuine et la fixation de l'asialofétuine ont été réalisées dans des conditions expérimentales identiques de façon à obtenir sur les gels des densités de ligand comparables. Une étude comparative de la capacité des gels obtenus a été réalisée. Les résultats sont résumés dans le tableau suivant dans lequel on a attribué arbitrairement l'efficacité 100% pour le gel à l'asialofétuine.

| | Capacité relative du gel (%) |
|---|---|
| Fétuine-Sépharose-4B | 79 |
| Asialofétuine-Sépharose-4B | 100 |

Les valeurs indiquées sont le résultat de deux déterminations. Chaque expérience a été réalisée en parallèle sur les deux types de supports en les mettant en contact avec un même volume de surnageant de culture contenant de la toxine pertussis. La quantité de toxine pertussis éluée a été déterminée par un dosage de protéine et par une méthode immunoenzymatique (ELISA).

**Revendications**

1. Utilisation comme ligand de chromatographie d'affinité pour la toxine pertussis, d'une glycoprotéine désialylée choisie parmi la fétuine désialylée et l'haptoglobine désialylée.

2. Utilisation selon la revendication 1, caractérisée par le fait que ladite protéine est l'asialofétuine.

3. Utilisation selon la revendication 1, caractérisée par le fait que ladite protéine est l'asialohaptoglobine.

4. Utilisation selon l'une quelconque des revendications précédentes, caractérisée par le fait que ladite protéine désialylée est fixée sur un support solide.

5. Procédé de purification des antigènes protéiques des bactéries du genre Bordetella, caractérisé par le fait qu'il consiste à mettre en contact une solution contenant lesdits antigènes protéiques avec un matériau de chromatographie d'affinité, puis à recueillir ou à éluer les antigènes recherchés, ledit matériau étant constitué par un support solide sur lequel est fixée une protéine désialylée choisie parmi la fétuine désialylée et l'haptoglobine désialylée.

6. Procédé selon la revendication 5, caractérisé par le fait que ladite protéine désialylée est fixée sur un support polymérique.

7. Procédé selon la revendication 6, caractérisé par le fait que ledit support polymérique est fixé sur un support minéral poreux.

8. Procédé selon l'une quelconque des revendications 5 à 7, caractérisé par le fait qu'après mise en contact de ladite solution avec le matériau de chromatographie, on élue la toxine pertussis.

9. Procédé selon l'une quelconque des revendications 5 à 7, caractérisé par le fait que la solution de départ est une fraction enrichie en F—HA, et qu'après mise en contact de ladite solution avec le matériau de chromatographie, on sépare une solution purifiée de F—HA débarrassée de toxine pertussis.

**Patentansprüche**

1. Verwendung eines von Sialinsäure befreiten Glykoproteins ausgewählt aus desialiniertem Fetuin und desialiniertem Haptoglobin als Ligand bei der Affinitätschromatographie von Keuchhustentoxin.

2. Verwendung nach Anspruch 1, dadurch gekennzeichnet, daß das Protein das Asialofetuin ist.

3. Verwendung nach Anspruch 1, dadurch gekennzeichnet, daß das Protein, das Asialohaptoglobin ist.

4. Verwendung nach einem der vorhergehenden Ansprüche, dadurch gekennzeichnet, daß das desialinierte Protein auf einem festen Trägermaterial fixiert ist.

5. Verfahren zur Reinigung von proteinischen

Antigenen von Bakterien des Stamms Bordetella, dadurch gekennzeichnet, daß es darin besteht, daß eine Lösung, welche die proteinischen Antigene enthält, mit einem chromatographischen Material mit einer Affinität dazu in Kontakt gebracht wird und anschließend die gewünschten Antigene gesammelt oder eluiert werden, wobei das Material aus einem festen Trägermaterial besteht, auf dem ein desialiniertes Protein, ausgewählt aus desialiniertem Fetuin und desialiniertem Haptoglobin fixiert ist.

6. Verfahren nach Anspruch 5, dadurch gekennzeichnet, daß das desialinierte Protein auf einem polymeren Trägermaterial fixiert ist.

7. Verfahren nach Anspruch 6, dadurch gekennzeichnet, daß das polymere Trägermaterial auf einem porösen mineralischem Trägermaterial fixiert ist.

8. Verfahren nach den Ansprüchen 5 bis 7, dadurch gekennzeichnet, daß man das Keuchhustentoxin eluiert, nachdem man die Lösung mit dem chromatographischen Material in Kontakt gebracht hat.

9. Verfahren nach den Ansprüchen 5 bis 7, dadurch gekennzeichnet, daß man eine Ausgangslösung verwendet, die eine Fraktion ist, welche mit F—HA angereichert ist und daß man nach dem in-Kontakt-bringen der gesamten Lösung mit dem chromatographischen Material eine gereinigte F—HA Lösung abtrennt, die vom Keuchhustentoxin befreit ist.

## Claims

1. Use of a desialylated glycoprotein selected from desialylated fetuin and desialylated haptoglobin as an affinity chromatography ligand for pertussis toxin.

2. Use according to Claim 1, characterized in that the said protein is asialofetuin.

3. Use according to Claim 1, characterized in that the said protein is asialohaptoglobin.

4. Use according to any one of the preceding claims, characterized in that the said desialylated protein is bound to a solid support.

5. Process for the purification of protein antigens of bacteria of the genus Bordetella, characterized in that it consists in bringing a solution containing the said protein antigens into contact with an affinity chromatography material, and then in collecting or eluting the desired antigens, the said material consisting of a solid support to which a desialylated protein selected from desialylated fetuin and desialylated haptoglobin is bound.

6. Process according to Claim 5, characterized in that the said desialylated protein is bound to a polymeric support.

7. Process according to Claim 6, characterized in that the said polymeric support is bound to a porous inorganic support.

8. Process according to any one of Claims 5 to 7, characterized in that, after the said solution is brought into contact with the chromatography material, the pertussis toxin is eluted.

9. Process according to any one of Claims 5 to 7, characterized in that the starting solution is a fraction enriched in F—HA and in that, after the said solution is brought into contact with the chromatography material, a purified solution of F—HA freed from pertussis toxin is separated.